# EUROPÄISCHE PATENTSCHRIFT

(11) **EP 0 340 562 B1**
(45) Veröffentlichungstag und Bekanntmachung des Hinweises auf die Patenterteilung: **13.04.1994**
(21) Anmeldenummer: 89107209.2
(22) Anmeldetag: 21.04.1989
(51) Int. Cl.: B01L 3/00

(54) **Testelement für Agglutinationsuntersuchungen, Verfahren zu seiner Herstellung und dessen Verwendung**
Testing device for agglutination analyses, its manufacturing method and use
Elément de test pour analyse d'agglutination, méthode de fabrication et utilisation

(30) Priorität: 29.04.1988 DE 3814585
(43) Veröffentlichungstag der Anmeldung: 08.11.1989
(73) Patentinhaber: F. HOFFMANN-LA ROCHE AG, 4002 Basel (CH)
(72) Erfinder: Schulz, Peter, Dr., D-8473 Pfreimd (DE)
(74) Vertreter: Schmidt, Horst, Dr.

(56) Entgegenhaltungen:
- EP-A- 0 212 314
- FR-A- 2 325 920
- US-A- 4 596 695
- ZENTRALVERBAND DER ELEKTROTECHNISCHEN INDUSTRIE B.V.,FÜGEN VON FORMTEILEN U. HALBZEUGEN AUS THERMOPLASTISCHEN KUNSTSTOFFEN MIT ULTRASCHALL, 1985, Seiten 17-22, Fachverband Elektroschweissgeräte, Frankfurt, DE; "Konstruktive Gestaltung der Formteile"

## Beschreibung

Die Erfindung betrifft ein Testelement für Agglutinationsuntersuchungen von einem Probe-Reagenz-Gemisch, welches Testelement wenigstens ein Paar plattenförmiger Körper enthält, zwischen denen eine Reaktionskapillare ausgebildet ist,
wobei der Querschnitt der Reaktionskapillare eine Grösse hat, die eine Strömung des Probe-Reagenz-Gemisches durch die Reaktionskapillare ermöglicht,
wobei die Reaktionskapillare einen aufstromseitigen Bereich und einen abstromseitigen Bereich enthält, an ihrem Aufstromende einen Eingang und an ihrem Abstromende einen Ausgang hat, und eine im wesentlichen rechteckförmige Querschnittskonfiguration in einer zur Längsachse der Reaktionskapillare senkrechten Richtung hat, und
wobei der Eingang der Reaktionskapillare in Fluidverbindung mit einem Bereich zur Aufnahme und Mischung der Probe mit einem Reagenz steht.

Die Erfindung betrifft ferner die Verwendung eines erfindungsgemässen Testelementes für immunologische Untersuchungen zum Nachweis von Stoffen, wie Arzneimitteln, Suchtmitteln oder dergleichen, bzw. einem Metaboliten des gesuchten Stoffes in Körperflüssigkeiten.

Der Nachweis bestimmter Stoffe, wie Arzneimittel, Suchtmittel, Hormone, virale, bakterielle und parasitäre Antigene, sowie von Stoffen, welche für das Vorliegen bestimmter Krankheiten indikativ sein können (z.B. Tumormarker) und dgl. in Körperflüssigkeiten, wie Urin oder Blut, kann anhand von Agglutinationsreaktionen erfolgen, die in einer Mischung aus einem Antikörperreagenz, einem Reagenz mit mit einem gesuchten Stoff bzw. einem Metaboliten des gesuchten Stoffes beschichteten Latex und der Körperflüssigkeit stattfinden. Da der nur in begrenzter Menge im Antikörperreagenz enthaltene Antikörper sich sowohl mit dem Stoff bzw. Metaboliten des Latex als auch mit dem Stoff der Körperflüssigkeit verbinden kann, werden Agglutinationen nur gebildet, wenn infolge Fehlens des gesuchten Stoffes bzw. des Metaboliten in der Körperflüssigkeit eine ausreichende Menge Antikörper zur Reaktion mit den Latexpartikeln vorhanden ist, so dass sich mehrere Latexpartikel zu visuell definierbaren Agglutinationen zusammenballen können. In Gegenwart des gesuchten Stoffes bzw. des Metaboliten in der Körperflüssigkeit stehen dagegen für die Zusammenballung wesentlich weniger Antikörper zur Verfügung, da die Antikörper mit dem in der Körperflüssigkeit vorhandenen gesuchten Stoff bzw. Metaboliten davon einen Immunkomplex bilden, so dass keine oder eine nur sehr schwache Agglutinationsreaktion stattfinden kann.

Eine andere Art des Nachweises kann anhand von Agglutinations reaktionen erfolgen, die in einer Mischung aus einem Reagenz mit mit Antikörpern gegen den nachzuweisenden Stoff beschichtetem Latex und der Körperflüssigkeit unter Umständen stattfinden. Bei Vorliegen einer ausreichenden Menge des gesuchten Stoffes in der Körperflüssigkeit bewirkt diese eine visuell definierbare Agglutination, während sie beim Fehlen desselben ausbleibt. Auf ähnliche Weisen können auch Antikörper im Blut oder anderen Körperflüssigkeiten nachgewiesen werden, wobei als Bindungspartner im Agglutinationssystem die entsprechenden Stoffe oder Antigene, oder aber auch Antikörper, die gegen die gesuchten Antikörper gerichtet sind, Verwendung finden.

Eine Vorgehensweise für derartige immunologische Untersuchungen besteht darin, dass die Probe-Reagenzien-Mischung in ein Proberöhrchen eingegeben und von Hand eine Zeitdauer lang durch Rütteln und Schwenken des Röhrchens vermischt und in Reaktion gehalten wird. Diese Vorgehensweise kann zu unterschiedlichen Agglutinationsreaktionen führen, je nachdem, über welche Zeitdauer und mit welcher Intensität das Rütteln und Schwenken des Proberöhrchens durchgeführt wird. In Anbetracht der damit verbundenen Unsicherheit bei der Auswertung der Reaktionsergebnisse wurde schon vorgeschlagen (vergl. DE-A-35 37 734 bzw. US-A-45 96 695), die Agglutinationsreaktion in einer Reaktionskapillare stattfinden zu lassen. Dadurch kann auf ein manuelles Schleudern und Rütteln der Probe-Reagenzien-Mischung verzichtet werden, da die durch die Kapillarwirkung hervorgerufene Bewegung der Mischung längs der Reaktionskapillare sichert, dass eine für die Bildung von Agglutinationen geeignete Durchmischung der Probe mit den Reagenzien und eine fortgesetzte Bewegung der Probe-Reagenzien-Mischung über eine gewisse Zeitdauer stattfindet.

Die Reaktionskapillare dieser bekannten Testvorrichtung ist zwischen zwei transparenten Glasplatten vorgesehen, die unter Bildung einer Kapillarkammer in einem bestimmten konstanten Abstand voneinander gehalten sind. Längs der Länge der Kapillarkammer ist daher der Kapillarwirkung für die jeweilige Probe-Reagenzien-Mischung im wesentlichen konstant. Dies hat den Nachteil, dass unter diesen Umständen die Reaktionsempfindlichkeit und die Reproduzierbarkeit der Reaktionsergebnisse starken Schwankungen unterliegen. Diese sind die Folge davon, dass die Probe-Reagenzien-Mischung bei verschiedenen Durchführungen des gleichen Tests unterschiedlich lange Zeit benötigt, um durch die Kapillarkammer zu strömen. Ein wesentliches Kriterium zur Erzielung reproduzierbarer Ergebnisse ist daher die Einhaltung gleichbleibender Reaktionszeiten. Eine weitere Voraussetzung für eine optimale Empfindlichkeit der Agglutinationsreaktion ist, dass für die Reaktion eine bestimmte Zeitdauer von z.B. 3 Minuten mit nur geringen Abweichungen nach oben und nach unten zur Verfügung steht.

Aus der EP-A-0 212 314 ist ein Testelement mit Reaktionskapillaren bekannt. Die EP-A-0 212 314 beschreibt u.a. eine Reaktionskapillare mit kreisförmigen Querschnitt, wobei der Radius entlang der Reaktionskapillare stufenweise variiert.

Der Erfindung liegt die Aufgabe zugrunde, ein einfach handhabbares Testelement der eingangs erwähnten Gattung zu schaffen, das bei unkompliziertem Aufbau und einer wirtschaftlichen Fertigungsmöglichkeit den Ablauf einer aussagekräftigen Agglutinationsreaktion in der jeweiligen Probe-Reagenzien-Mischung innerhalb einer reproduzierbaren Zeitdauer ohne Einwirken äusserer Kräfte, wie manuelle Rüttel- oder Schleuderbewegungen oder thermische Konvektion, ermöglicht.

Diese Aufgabe wird erfindungsgemäss mit einem Testelement der eingangs genannten Art gelöst, das dadurch gekennzeichnet ist, dass im aufstromseitigen Bereich bzw. im abstromseitigen Bereich die Abmessung der Reaktionskapillare in Dickenrichtung vom Aufstromende zum Abstromende jedes dieser Bereiche zunimmt, wobei die Zunahme dieser Abmessung stetig und linear über die ganze Länge jeder dieser Bereiche ist.

In einem erfindungsgemässen Testelement wird daher im aufstromseitigen Bereich der Reaktionskapillare eine Pumpwirkung auf die Probe-Reagenzien-Mischung ausgeübt, was eine stetige Zufuhr und Bewegung der Mischung in und durch den abstromseitigen Bereich sicherstellt, der deshalb auf optimale Verhältnisse für die Bildung von Agglutinationen ausgelegt werden kann. Wie nachstehend beschrieben, fliesst die Probe-Reagenzien-Mischung vom aufstromseitigen zum abstromseitigen Bereich. Vorteilhafterweise unterliegt die Zeit, die eine Probe-Reagenzien-Mischung benötigt, um vom Aufstromende zum Abstromende der Reaktionskapillare zu fliessen, bei einer gegebenen Abmessung des Testelementes für eine bestimmte Testflüssigkeit zwischen verschiedenen Tests nur sehr geringen Schwankungen. Damit ist eine wichtige Bedingung für eine höchstmögliche Empfindlichkeit der Agglutinationsreaktion erfüllt. Die Sicherheit bei der Auswertung der Ergebnisse wird dadurch wesentlich erhöht. Da die Reaktionskapillare einen abstromseitigen Bereich hat, in dem die Strömungsgeschwindigkeit der Probe-Reagenzien-Mischung geringer als am aufstromseitigen Bereich ist, können größere Zusammenballungen von Latexpartikeln stattfinden, ohne dass der Fluß der Probe-Reagenzien-Mischung durch die Reaktionskapillare hierdurch wesentlich beeinträchtigt oder sogar unterbunden wird. Grössere Zusammenballungen von Latexpartikeln erleichtern andererseits die visuelle Testauswertung bzw. Handhabung des Testelementes in der Praxis.

Bevorzugt wird insbesondere eine Ausgestaltung des erfindungsgemässen Testelements, bei der der Querschnitt der Reaktionskapillare in der Durchflussrichtung der Probe-Reagenzien-Mischung kontinuierlich und linear über die ganze Länge des aufstromseitigen Bereiches bzw. des abstromseitigen Bereiches zunimmt.

Gemäss einer bevorzugten Weiterbildung der Erfindung ist die Abmessung des Querschnitts der Reaktionskapillare in Dickenrichtung (d.h. der Abstand zwischen den plattenförmigen Körpern) längs des aufstromseitigen Bereiches kleiner als längs des abstromseitigen Bereiches ist. Mithin herrschen am aufstromseitigen Bereich grössere Kapillarkräfte als am abstromseitigen Bereich, was entsprechend unterschiedliche Strömungsgeschwindigkeiten durch die Reaktionskapillare zur Folge hat.

Versuche haben gezeigt, daß die Einhaltung einer bestimmten Durchlaufzeit der Probe-Reagenzien-Mischung durch die Reaktionskapillare innerhalb enger Grenzen weiter dadurch gefördert wird, wenn gemäss einer anderen bevorzugten Weiterbildung der Erfindung die Reaktionskapillare einen im wesentlichen S-förmigen Verlauf hat, wobei die Reaktionskapillare zwischen dem aufstromseitigen Bereich und dem abstromseitigen Bereich ein einen Zwischenbereich enthält, der mit dem aufstromseitigen Bereich und dem abstromseitigen Bereich in Fluidverbindung steht, wobei im Zwischenbereich die Abmessung in Dickenrichtung von seinem Aufstromende zu seinem Abstromende stetig und linear über die ganze Länge des Zwischenbereiches zunimmt, und wobei am Aufstromende des Zwischenbereichs die Abmessung in Dickenrichtung wenigstens so gross wie die Abmessung in Dickenrichtung am Abstromende des aufstromseitigen Bereiches ist, und am Abstromende des Zwischenbereichs die Abmessung in Dickenrichtung höchstens so gross wie die Abmessung in Dickenrichtung am Aufstromende des abstromseitigen Bereiches ist.

Am Abstromende der Reaktionskapillare kann ein Bereich vorgesehen sein, in dem sich die Probe-Reagenzien-Mischung nach Durchlauf durch die Kapillare ansammelt. Dies erleichtert die Beobachtung und Auswertung der Agglutinationsreaktion.

Das Testelement setzt sich vorzugsweise aus in besonders wirtschaftlicher Weise herstellbaren Spritzgiessteilen zusammen, die aus einem geeigneten transparenten Kunststoffmaterial hergestellt werden. Das Testelement enthält durch Spritzgiessen gefertigte Ober- und Unterplatten, die durch Schweissen oder Kleben miteinander verbunden sind, und die zwischen sich die Reaktionskapillare vorsehen. Ein bevorzugtes Verfahren zur Herstellung des Testelementes sieht vor, dass die Verbindung der Ober- und Unterplatten unter Schaffung einer Reaktionskapillare der erfindungsgemässen Konfiguration durch Ultraschallschweissen erfolgt, indem man an einem der plattenförmigen Körper einen wenigstens längs der zu bildenden Reaktionskapillare sich erstreckenden Schweissteg und am anderen plattenförmigen Körper eine komplementäre Schweissnut mit einer Querschnittskonfiguration ausbildet, dass der Schweissteg darin nur teilweise aufnehmbar ist, die plattenförmigen Körper unter Eingriffnahme des Schweissteges und der Schweissnut zusammenbringt, und an die plattenförmigen Körper Ultraschallenergie einlegt, so dass die mit der Schweissnut in Berührung stehenden Bereiche des Schweissteges schmelzen und dieser unter Verbindung der plattenförmigen Körper in die Schweissnut bis zu einer vorgegebenen Tiefe eindringt, um eine Reaktionskapillare mit einer Abmessung in Dickenrichtung zu schaffen, die am Aufstromende kleiner als am Abstromende ist. Auf diese Weise kann die Abmessung der Reaktionskapillare in Dickenrichtung zur Erzielung der gewünschten unterschiedlichen Kapillarwirkung exakt eingehalten werden. Dabei zeichnet sich das Ultraschallschweissen durch eine besondere Wirtschaftlichkeit aus.

Bezüglich anderer Weiterbildungen der Erfindung wird auf die Patentansprüche verwiesen.

Ein anderer Aspekt der Erfindung ist die Verwendung eines Testelementes der vorerwähnten Art für immunologische Untersuchungen zum Nachweis von Suchstoffen, wie Arzneimitteln, Suchtmitteln und dgl., bzw. einem Metaboliten des gesuchten Stoffes in Körperflüssigkeiten. Diese Verwendung ist dadurch gekennzeichnet, dass
a) auf den Aufnahme- und Mischbereich des Testelementes nacheinander bestimmte Mengen der Körperflüssigkeit, eines Antikörperreagenz und eines Latexreagenz aufgegeben werden,
b) die Körperflüssigkeit, das Antikörperreagenz und das Latexreagenz miteinander vermischt werden, so dass diese ein Probe-Reagenz-Gemisch bilden,
c) man das Probe-Reagenz-Gemisch in die Reaktionskapillare ein ziehen lässt, und
d) nach Durchtritt des Gemisches durch die Reaktionskapillare das Vorhanden sein oder Fehlen des gesuchten Stoffes durch visuelle Beobachtung des Vorhandenseins bzw. Fehlen von Produkten von Agglutinationsreaktionen im Sammelbereich des Testelementes festgestellt wird.

Der Antikörper kann entweder Teil des Reagenz oder in der Körperflüssigkeit vorhanden sein, je nach dem ob ein kompetitives oder ein nichtkompetitives Nachweisverfahren durchführt wird.

Die Erfindung wird nachfolgend anhand einer Ausführungsform und der Zeichnung näher erläutert. Es zeigen:
- Fig. 1: in Draufsicht ein Testelement für Agglutinationsuntersuchungen gemäss einer bevorzugten Ausführungsform der Erfindung,
- Fig. 2-4: geschnittene Ansichten längs der Schnittlinien II-II, III-III und IV-IV in Fig. 1,
- Fig. 5: eine geschnittene Ansicht längs der Schnittlinie V-V in Fig. 1, und
- Fig. 6A,6B: fragmentarische quergeschnittene Ansichten in vergrössertem Massstab des Testelementes mit Darstellung der Lage der plattenförmigen Körper vor und nach deren Verschweissen.

Das Testelement nach der bevorzugten Ausführungsform der Erfindung trägt das allgemeine Bezugszeichen 1 und umfasst, wie dargestellt, ein Paar aufeinander angeordnete untere und obere plattenförmige Körper 2, 3, die längs nachfolgend noch näher erläuterten Stegbereichen 13, z.B. durch Kleben oder Schweissen, fest miteinander verbunden sind. An einem der plattenförmigen Körper 2 oder 3, insbesondere dem oberen Körper, sind Bereiche definiert, die der Aufnahme, Bewegung und Sammlung einer Probe-Reagenzienmischung für Agglutinationsreaktionen dienen, worauf ebenfalls nachfolgend noch näher eingegangen wird.

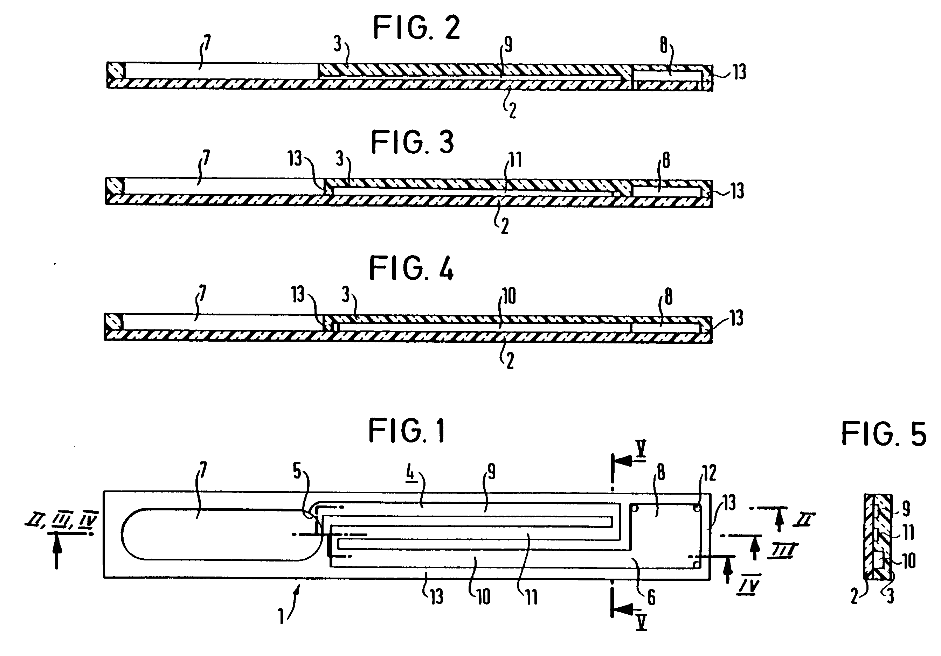

Insbesondere ist an einem in Fig. 1 linksseitigen Endabschnitt des Testelementes 1 eine längliche Aussparung vorgesehen, die den oberen plattenförmigen Körper 3 durchsetzt und nach unten unter Bildung eines Aufnahme- und Mischbereiches 7 für die Probe und die Agglutinationsreagenzien durch den unteren plattenförmigen Körper 2 fluiddicht abgedeckt ist. Am anderen rechtsseitigen Endabschnitt (d.h. im Sammelbereich) des Testelementes 1 ist in der Unterfläche des oberen plattenförmigen Körpers 3 eine im wesentlichen rechteck-, quadrat- oder andersförmige Ausnehmung definiert, die in Verbindung mit dem sie überdeckenden unteren plattenförmigen Körper 2 eine Kammer oder einen Bereich 8 definiert, in dem sich die Probe-Reagenzienmischnung nach Durchströmen einer zwischen den Bereichen 7 und 8 sich erstreckende im wesentlichen S-förmig verlaufenden Reaktionskapillare 4 zur weiteren Beobachtung ansammeln kann.

Die Reaktionskapillare 4 ist durch eine nutförmigen Ausnehmung in der Unterfläche des oberen plattenförmigen Körpers 3 gebildet, die fluiddicht durch den unteren plattenförmigen Körper 2 abgedeckt ist. Längs des Aufnahme- und Mischbereiches 7, der Reaktionskapillare 4 und des Sammelbereiches 8 erstreckt sich der Steg 13, der somit eine seitliche fluiddichte Begrenzung dieser Bereiche und der Reaktionskapillare bildet (siehe Fig. 2-4).

Die Reaktionskapillare 4 umfasst einen ersten oder aufstromseitigen Kapillarbereich 9, der an seinem einen oder Aufstromende 5 in Fluidverbindung mit dem Aufnahme- und Mischbereich 7 und an seinem anderen Ende mit dem einen aufstromseitigen Ende eines zweiten oder Zwischenbereiches 11 steht. Der Zwischenbereich 11 ist wiederum an seinem anderen Ende mit dem einen oder aufstromseitigen Ende eines dritten oder abstromseitigen Bereiches 10 verbunden. Das andere oder Abstromende 6 des Bereiches 10 steht in Fluidverbindung mit dem Sammelbereich 8. Die ersten, zweiten und dritten Kapillarbereiche 9, 10, 11 haben sämtlich vorzugsweise einen im wesentlichen rechteckförmigen Querschnitt, wie am besten aus Fig. 5 zu entnehmen ist, und erstrecken sich vorzugsweise parallel zueinander, wobei die Kapillarbereiche 9 und 11 bzw. 11 und 10 an deren Enden über dazu senkrecht liegenden Kapillarsegmente miteinander verbunden sind.

Die Abmessung des ersten und zweiten Kapillarbereiches 9, 11 in Breitenrichtung (d.h. der Abstand zwischen den Seitenwänden der Reaktionskapillare, wenn diese entlang ihrer längsachse betrachtet wird) ist bei der vorliegenden Ausführung im wesentlichen gleich und kleiner als die Abmessung des abstromseitigen Kapillarbereiches 10 in Breitenrichtung. Es versteht sich jedoch, dass die Erfindung weder auf die beschriebene Querschnittskonfiguration noch Breitenabmessung der Reaktionskapillare beschränkt ist. In Dickenrichtung, d.h. in Richtung senkrecht zur Ebene der Fig. 1 (siehe den Abstand zwischen den Körpern 2 und 3 in Figuren 2-4), unterscheiden sich wenigstens der aufstromseitige erste und der abstromseitige dritte Bereich 9, 10 voneinander. Insbesondere ist die Dickenabmessung des abstromseitigen Bereiches 10 grösser als die des aufstromseitigen Bereiches 9. Der zwischenliegende Bereich 11 kann eine Dickenabmessung zwischen der des aufstromseitigen und der des abstromseitigen Bereiches 9, 10 haben. In der bevorzugten Ausführungsform hat jede Stelle im abstromseitigen Kapillarbereich eine Querschnittsfläche, die grösser als oder gleich wie diejenige einer anderen Stelle ist, die sich in Bezug auf die erstgenannte Stelle im aufstromseitigen Kapillarbereich befindet.

Infolge der unterschiedlichen Dickenabmessung der Kapillarbereiche 9, 10 und 11 werden darin unter der Voraussetzung ansonsten gleicher übriger kapillarwirksamer Einflussfaktoren unterschiedliche Kapillarkräfte hervorgerufen und ist insbesondere die Kapillarkraft im aufstromseitigen Bereich 9 grösser als am abstromseitigen Bereich 10. Die entstehenden Kapillarkräfte im Zwischenbereich 11 können durch geeignete Wahl der Dickenabmessung dieses Bereiches so eingestellt werden, dass sie gleich oder kleiner als die Kapillarkräfte im aufstromseitigen Bereich 9 und/oder gleich oder grösser als im abstromseitigen Bereich 10 sind.

Wie insbesondere aus Fig. 2-4 zu entnehmen ist, ist die Dickenabmessung längs der Kapillarbereiche 9, 10, 11 von jeweils deren Auf- zu den Abstromenden nicht konstant. Sie ändert sich vielmehr vorzugsweise stetig, wenn erwünscht, auch stufenförmig, wobei die Dickenabmessung an den aufstromseitigen Enden der Kapillarbereichen jeweils geringer als an deren abstromseitigen Enden ist. Jeder Kapillarbereich 9, 10, 11 der Reaktionskapillare 4 hat vorzugsweise in Längsrichtung (d.h in Durchflussrichtung) eine im wesentlichen konische oder keilförmige Querschnittskonfiguration. Dagegen kann, wie dargestellt, die Dickenabmessung des Sammelbereiches 8 am Abstromende 6 der Reaktionskapillare 4 konstant sein. Mit dem Bezugszeichen 12 in Fig. 1 sind Ventilationsbohrungen zur Entlüftung des Sammelbereiches 8 angedeutet.

Die infolge der unterschiedlichen Querschnittsausbildung, insbesondere Dickenabmessung, längs der Reaktionskapillare 4 hervorgerufenen unterschiedlichen Kapillarkräfte bewirken, dass die Strömungsgeschwindigkeit der Probe-Reagenzienmischung längs des aufstromseitigen Kapillarbereiches 9 grösser als längs des abstromseitigen Kapillarbereiches 10 ist, während längs des zwischenliegenden Kapillarbereiches 11 Uebergangsverhältnisse herrschen können. Die höheren Kapillarkräfte im aufstromseitigen Kapillarbereich 9 haben eine kräftige stetige einziehende Wirkung auf die im Aufnahme- und Mischbereich 7 befindlichen Reagenzien in die Reaktionskapillare 4 zur Folge, und die höhere Strömungsgeschwindigkeit längs dieses Kapillarbereiches bewirkt, dass die Probe-Reagenzienmischung in die nachfolgenden Kapillarbereiche 10, 11, wo eine geringere Kapillarwirkung herrscht, "hineingepumpt" wird. Es wird hierdurch eine positive Strömung der Probe-Reagenzienmischung längs der gesamten Reaktionskapillare 4 und in den Sammelbereich 8 erzielt und aufrechterhalten.

Die Abmessungen der Reaktionskapillare 4 in Dickenrichtung von deren Auf- zum Abstromende 5 bzw. 6 sind grundsätzlich auf die jeweilige zu untersuchende Probe-Reagenzienmischung abzustimmen. Je nach Mischung kann die Dickenabmessung am Aufstromende 5 zwischen 1 und 100 µm und am Abstromende 6 zwischen 5 und 500 µm betragen. Vorzugsweise ist die Reaktionskapillare am Aufstromende 5 zwischen 50 und 100 µm und am Abstromende 6 zwischen 200 und 300 µm dick.

Andere Dickenabmessungen können auch vorgesehen werden und die Dickenabmessung der Reaktionskapillare längs deren Länge muss sich nicht stets von einem Bereich geringer Dicke zu einem Bereich grösserer Dicke in Durchströmungsrichtung ändern. In einer bevorzugten Ausführungsform nimmt jedoch die Querschnittsfläche der Reaktionskapillare kontinuierlich und stetig zu (z.B. in Form einer linearen Zunahme), vom aufstromseitigen zum abstromseitigen Ende und über jede der Kapillarbereiche 9, 11 und 10. Als Alternative können, wenn erwünscht, auch zwischenliegende Bereiche mit geringerer solchen mit grösserer Strömungsgeschwindigkeit vorgelagert sein.

Ferner ist darauf hinzuweisen, dass die Kapillarwirkung bzw. die Strömungsgeschwindigkeit längs der Reaktionkapillare auch durch andere Faktoren als die Querschnittsgestaltung beeinflusst bzw. verändert werden kann. Ohne Anspruch auf Vollständigkeit sind dies insbesondere die Oberflächenbeschaffenheit, insbesondere Rauhigkeit der Kapillarwände, das Material, aus dem das Testelement gebildet ist, insbesondere darin enthaltene Zusätze, wie Additive oder Feuchtigkeit, innerere Spannungen und Materialorientierungen aus der Herstellung des Testelementes und dgl..

Vorzugsweise setzt sich das Testelement aus Spritzgiessteilen zusammen, die aus einem geeigneten transparenten Kunststoffmaterial wie z.B. Polycarbonate, Polystyrol, Polymethylmethacrylat oder Harze auf Basis von Acrylnitril-Butadien-Styrol hergestellt werden. Besonders bevorzugt wird wegen seiner guten optischen Eigenschaften, guten Schweissbarkeit und Verarbeitbarkeit im Spritzgiessverfahren Polymethylmethacrylat (PMMA). Es versteht sich jedoch, dass, wenn erwünscht, auch andere Materialien, einschliesslich anorganischer Materialien, wie Glas, verwendet werden können. Es ist ausserdem vorgesehen, Harze zu verwenden, die antistatische Materialien als Additive enthalten. Zur Unterdrückung der statischen Ladung ist es vorgesehen, einen Kunststoff mit Russ als Additiy zu verwenden.

Bei Verwendung von Polymethylmethacrylat (PMMA) kann z.B. einer der folgenden Additive verwendet werden:
a) "Carbon Black Pigment" der Firma Degussa, Hanau, BRD, mit der Handelsbezeichnung Masterbatch CB 51. Dieser Additiv wird zu einem Prozenzsatz von 3-5 % hinzugegeben.
b) Ein Antistatikum der Firma ICI mit der Handelsbezeichnung ATMER 129, welches Glycerinmonostereat (90 %) und einige Glycerindistereate enthält. Dieser Additiv wird zu Prozentsätzen von z.B. 0,125 %, 0,25 % oder 0,5 % hinzugegeben.
c) Ein Antistatikum der Firma Lanko, U.K., mit der Handelsbezeichnung LANKOSTAT JP, welches Laurinsäurediethanolamin enthält. Dieser Additiv wird z.B. zu einem Prozentsatz von 0,5 % hinzugegeben.

Ferner versteht es sich, dass anstelle einer Reaktionskapillare 4 mit im wesentlichen parallel zueinander verlaufenden Kapillarbereichen auch eine geradlinige Erstreckung der Reaktionskapillare oder jede andere beliebige Konfiguration, z.B. spiralförmige Anordnung, der Kapillarbereiche, vorgesehen werden kann. Das Vorsehen eines Kapillarzwischenbereiches 11 ist nicht obligatorisch. Im Rahmen der Erfindung ist es ausserdem vorgesehen, dass die bei Verwendung des Testelementes für ein kompetitives Nachweisverfahren die Probe-Reagenzienmischung einen Antikörper enthält, was bei einem nichtkompetitiven Nachweisverfahren nicht nötig ist. In einem nichtkompetitiven Nachweisverfahren ist der Antikörper in der Körperflüssigkeit enthalten (z.B. bei AIDS-Tests).

Unter Verwendung eines Testelementes der vorbeschriebenen Art kann eine Agglutinationsuntersuchung z.B. wie folgt erfolgen. Auf den Aufnahme- und Mischbereich 7 des Testelementes 1 wird nacheinander eine bestimmte Menge der zu untersuchenden Flüssigkeit, z. B. des Urins oder Blutes einer Person, zusammen mit bestimmten Mengen, z.B. je eines Tropfens, eines Antikörper enthaltenden Reagenzes, eines Pufferreagenzes und eines Reagenzes mit mit einem gesuchten Stoff bzw. einem Metaboliten des gesuchten Stoffes beschichteten Latexpartikeln aufgegeben und anschliessend auf dem Aufnahme- und Mischbereich 7 leicht miteinander vermischt. In einem kompetitiven Nachweisverfahren enthält das Reagenz auch Antikörper. Infolge der starken Kapillarwirkung am aufstromseitigen Kapillarbereich 9 wird die Mischung durch die Öffnung 5 in die Reaktionskapillare 4 selbsttätig eingezogen und in die nachfolgenden Kapillarbereiche 10 und 11 "gepumpt".

Ein agglutinationsaktiver Zustand des Probe-Reagenziengemisches wird alleine durch deren Strömung längs der Reaktionskapillare aufrechterhalten, so dass es keiner äusserer Kräfte auf das Testelement bedarf, um die Mischung in Bewegung zu halten. Die Bildung sichtbarer Agglutinationen erfolgt im wesentlichen längs des abstromseitigen Kapillarbereiches 10 und kann insbesondere am Sammelbereich 8 visuell gut beobachtet und ausgewertet werden. Infolge der Pumpwirkung des aufstromseitigen Kapillarbereiches 9 ist die Zeit, die für die Strömung des Probe-Reagenziengemisches vom Auf-zum Abstromende 5 bzw. 6 der Reaktionskapillare 4 bzw. bis zur vollständigen Füllung des Sammelbereiches 8 verstreicht, gut reproduzierbar und kann insbesondere auf das jeweilige Probe-Reagenziengemisch zur Erzielung maximaler Empfindlichkeit optimiert werden.

Das Testelement eignet sich insbesondere zum Nachweis von Kokainmetaboliten. Hierzu wird z.B. ein mit Benzoylecgonin beschichteter Latex, ein monoklonaler Antikörper gegen Benzoylecgonin sowie Urin als Körperflüssigkeit verwendet. Die Erfindung ist jedoch auf eine derartige Verwendung nicht beschränkt.

### Beispiel

Es wurde ein Testelement nach Fig. 1 unter Verwendung eines Polymethylmethacrylat-Kunststoffes (PMMA), z.B. derjenige, der unter dem Handelsnamen Diacon bezogen werden kann, durch Spritzgiessen und Ultraschallschweissen nach einem nachfolgend näher beschriebenen Verfahren hergestellt. Die Abmessungen der Reaktionskapillare betrugen:

Länge der Kapillarbereiche 9,11,10: jeweils ca. 50 mm; Breite des aufstrommseitigen und zwischenliegenden Kapillarbereiches 9,11: jeweils ca. 2 mm; Breite des abstromseitigen Kapillarbereiches 10: 3 mm; Dickenabmessung der Kapillarbereiche, gemessen jeweils an deren Aufstrom- und Abstromenden: aufstromseitiger Kapillarbereich 9: 80 bzw. 130 µm, zwischenliegender Kapillarbereich 11: 130 bzw. 170 µm, abstromseitiger Kapillarbereich 10: 200 bzw. 240 µm; Dicke des Sammelbereiches: 240 µm.

Eine Probe-Reagenzienmischung der vorerwähnten Art zum Nachweis von Suchtmitteln in Urin wurde auf den Aufnahmeund Mischbereich 7 des Testelementes aufgegeben und die Zeit gemessen, die für die Bewegung der Probe-Reagenzienmischung längs der einzelnen Kapillarbereiche bis zur vollständigen Füllung des Sammelbereiches 8 verstreicht. Es wurden folgende Durchschnittszeiten ermittelt: 12 Sek. für den aufstromseitigen Kapillarbereich 9, 26 Sek. für den Zwischenbereich 11 und 140 Sek. für den abstromseitigen Kapillarbereich 10 einschliesslich des Sammelbereiches 8. Insgesamt ergab sich eine Zeit von etwa 3 Min. für die Agglutinationsreaktion, die als optimal für die betreffende Reagenzienmischung hinsichtlich der Auswertungsempfindlichkeit angesehen wird. Der Variationskoeffizient der Reaktionszeit ([Standardabweichung/Mittelwert] x 100) für irgend einen Satz von Messwerten sollte vorzugsweise nicht mehr als ± 10 % sein.

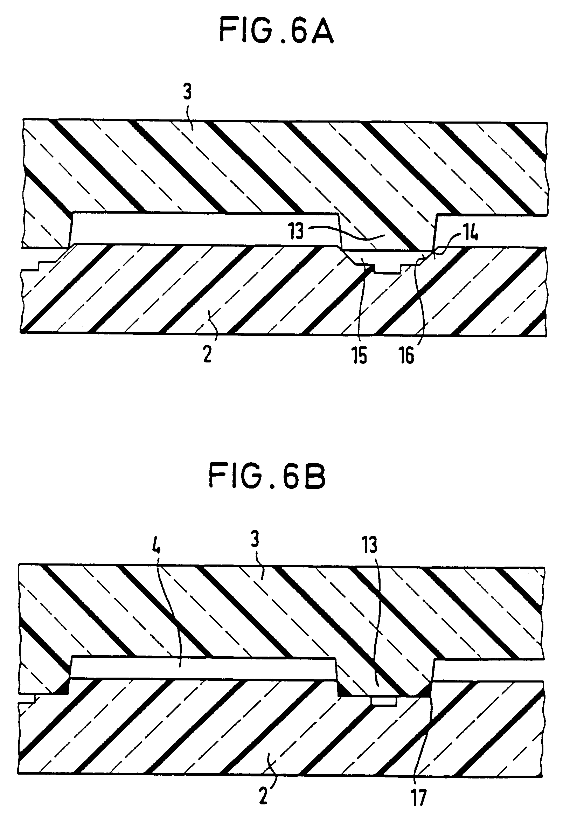

Nachfolgend wird ein bevorzugtes Verfahren zum Verbinden der durch Spritzgiessen eines Kunststoffmaterials, insbesondere PMMA, hergestellten plattenförmigen Körper 2, 3 anhand von Fig. 6A, 6B beschrieben. Fig. 6A und 6B zeigen die plattenförmigem Körper 2, 3 im Zustand vor bzw. nach dem Verbinden durch Ultraschallschweissen. Wie eingangs beschrieben, erstreckt sich umfänglich längs der Bereiche 7 und 8, wenigstens aber längs der Reaktionskapillare 4 ein durchgehender (wenn erwünscht auch unterbrochener) Steg 13. Der Steg 13 hat in der Ausgangsposition nach Fig. 6A einen im wesentlichen rechteck- oder leicht trapezförmigen Querschnitt. Dem Steg 13 am oberen plattenförmigen Körper 3 entspricht eine komplementäre Nut 15 im unteren plattenförmigen Körper 2 mit einer Querschnittkonfiguration, dass der Steg 13, wie dargestellt, darin nur teilweise aufgenommen werden kann. Insbesondere hat die Nut 15 einen im wesentlichen kegelstumpfförmigen nach innen sich verjüngenden Querschnitt, so dass die seitlichen Kanten 14 des Steges 13 in Eingriff in mit einer zwischenliegenden Stelle der konischen Seitenwände 16 der Nut 15 treten können. Wird in dieser Ausgangsstellung in dem Fachmann bekannter Weise Ultraschallenergie an die plattenförmigen Körper 2, 3 angelegt, so schmelzen die Bereiche des Steges 13, die mit den Seitenwänden 16 der Nut 15 in Eingriff stehen, wie dies in Fig. 6B bei 17 angedeutet ist.

Unter dem auf die plattenförmigen Körper 2, 3 ausgeübten Schweissdruck wird sich daher der Steg 13 weiter in die Nut 15 hineinbewegen, bis die Stirnfläche des Steges 13 in Anlage mit der Bodenfläche der Nut 15 kommt. In diesem Stadium ist nicht nur die Dickenabmessung der zwischen den benachbarten Stegbereichen liegenden Reaktionskapillare 4 und des Sammelbereiches 8 festgelegt, sondern werden diese Bereiche auch fluiddicht nach aussen abgedichtet und kommt eine feste Verbindung der plattenförmigen Körper 2, 3 zustande. Der unterschiedlichen Dickenabmessung der Reaktionskapillare 4 längs ihrer longitudinalen Erstreckung kann entweder durch entsprechende Formgestaltung, insbesondere Tiefe der die Reaktionskapillare bildenden Flächenabschnitte des oberen und/oder unteren plattenförmigen Körpers Rechnung getragen werden. Als Alternative hierzu können besagte Flächenabschnitt auch mit konstanter Tiefe gespritzt werden, während beim Schweissen die plattenförmigen Körper 2, 3 in einer nicht gezeigten schrägen Ausgangsbeziehung zueinander gehalten werden. Dies hat zur Folge, dass ein Teil, in Fig. 1 der linksseitige Teil, des Steges 13, eine andere Lage in der zugehörigen Nut 15 einnimmt wie der andere rechtsseitige Teil des Steges. Wird unter diesen Umständen eine Ultraschallenergie an die plattenförmigen Körper 2, 3 angelegt, so wird sich der linksseitige Stegteil unter dem Schweissdruck tiefer als der rechtsseitige Stegteil in die Nut 15 unter entsprechender unterschiedlicher Querschnitts- ausbildung der Reaktionskapillare 4 bewegen. Es versteht sich, dass die Anordnung des Schweissteges und der Schweissnut auch umgekehrt sein kann, indem ersterer am unteren und letztere am oberen plattenförmigen Körper ausgebildet ist.

Die Erfindung wurde vorausgehend anhand einer bevorzugten Ausführungsform beschrieben. Es versteht sich, dass die Erfindung hierauf nicht beschränkt ist. Insbesondere kann das erfindungsgemässe Prinzip der Ausbildung einer Agglutinationskapillare auch bei Testeinrichtungen beliebiger anderer Art angewandt werden. Ferner kann die unterschiedliche Kapillarwirkung der auf- und abstromseitigen Bereiche der Reaktionskapillare nicht nur durch eine unterschiedliche Dickenabmessung, sondern generell durch unterschiedliche Querschnittsabmessungen der Kapillarbereiche erzielt werden. Der Ausdruck "Reaktionskapillare" schliesst in Bezug auf den abstromseitigen Bereich auch keine oder eine nur sehr geringe Kapillarwirkung ein.

## Patentansprüche

1. Testelement für Agglutinationsuntersuchungen von einem Probe-Reagenz-Gemisch, welches Testelement wenigstens ein Paar plattenförmiger Körper (2, 3) enthält, zwischen denen eine Reaktionskapillare (4) ausgebildet ist,
wobei der Querschnitt der Reaktionskapillare (4) eine Grösse hat, die eine Strömung des Probe-Reagenz-Gemisches durch die Reaktionskapillare ermöglicht,
wobei die Reaktionskapillare (4) einen aufstromseitigen Bereich (9) und einen abstromseitigen Bereich (10) enthält, an ihrem Aufstromende (5) einen Eingang und an ihrem Abstromende (6) einen Ausgang hat, und eine im wesentlichen rechteckförmige Querschnittskonfiguration in einer zur Längsachse der Reaktionskapillare senkrechten Richtung hat, und
wobei der Eingang der Reaktionskapillare (4) in Fluidverbindung mit einem Bereich (7) zur Aufnahme und Mischung der Probe mit einem Reagenz steht,
dadurch gekennzeichnet, dass
im aufstromseitigen Bereich (9) bzw. im abstromseitigen Bereich (10) die Abmessung der Reaktionskapillare (4) in Dickenrichtung vom Aufstromende zum Abstromende jedes dieser Bereiche (9, 10) zunimmt, wobei die Zunahme dieser Abmessung stetig und linear über die ganze Länge jeder dieser Bereiche (9, 10) ist.

2. Testelement gemäss Anspruch 1, dadurch gekennzeichnet, dass die Abmessung der Reaktionskapillare (4) in Dickenrichtung an ihrem Aufstromende (5) zwischen 1 und 100 µm und an ihrem Abstromende (6) zwischen 5 und 500 µm liegt, und dass die kleinste Abmessung der Reaktionskapillare (4) in Dickenrichtung im abstromseitigen Bereich (10) wenigstens so gross wie die grösste Abmessung der Reaktionskapillare (4) in Dickenrichtung im aufstromseitigen Bereich (9) ist.

3. Testelement gemäss Anspruch 2, dadurch gekennzeichnet, dass die Abmessung der Reaktionskapillare (4) in Dickenrichtung an ihrem Aufstromende (5) zwischen 50 und 100 µm und an ihrem Abstromende (6) zwischen 200 und 300 µm liegt.

4. Testelement gemäss Anspruch 1, dadurch gekennzeichnet, dass die Reaktionskapillare (4) zwischen dem aufstromseitigen Bereich (9) und dem abstromseitigen Bereich (10) einen Zwischenbereich (11) enthält, der mit dem aufstromseitigen Bereich und dem abstromseitigen Bereich in Fluidverbindung steht, wobei im Zwischenbereich (11) die Abmessung in Dickenrichtung von seinem Aufstromende zu seinem Abstromende stetig und linear über die ganze Länge des Zwischenbereiches (11) zunimmt, und wobei am Aufstromende des Zwischenbereichs (11) die Abmessung in Dickenrichtung wenigstens so gross wie die Abmessung in Dickenrichtung am Abstromende des aufstromseitigen Bereiches (9) ist, und am Abstromende des Zwischenbereichs (11) die Abmessung in Dickenrichtung höchstens so gross wie die Abmessung in Dickenrichtung am Aufstromende des abstromseitigen Bereiches (10) ist.

5. Testelement gemäss Anspruch 1, dadurch gekennzeichnet, dass jeder der plattenförmigen Körper (2, 3) eine bestimmte Länge hat und die Reaktionskapillare (4) eine Länge hat die grösser als die Länge eines der plattenförmigen Körper ist.

6. Testelement gemäss Anspruch 1 , dadurch gekennzeichnet, dass die Reaktionskapillare (4) einen im wesentlichen S-förmigen Verlauf hat.

7. Testelement nach einem der vorhergehenden Ansprüche, dadurch gekennzeichnet, dass es ferner einen Sammelbereich (8) enthält, der in Fluidverbindung mit dem Ausgang (6) der Reaktionskapillare (4) steht.

8. Testelement nach einem der vorhergehenden Ansprüche, dadurch gekennzeichnet, dass es sich aus Spritzgiessteilen zusammensetzt, die aus einem transparenten Kunststoffmaterial hergestellt sind.

9. Verwendung eines Testelementes nach einem der Ansprüche 1 bis 8 für immunologische Untersuchungen zum Nachweis von Stoffen, wie Arzneimitteln, Suchtmitteln oder dergleichen, bzw. einem Metaboliten des gesuchten Stoffes in Körperflüssigkeiten, dadurch gekennzeichnet, dass
a) auf den Aufnahme- und Mischbereich (7) des Testelementes nacheinander bestimmte Mengen der Körperflüssigkeit, eines Antikörperreagenz und eines Latexreagenz aufgegeben werden,
b) die Körperflüssigkeit, das Antikörperreagenz und das Latexreagenz miteinander vermischt werden, so dass diese ein Probe-Reagenz-Gemisch bilden,
c) man das Probe-Reagenz-Gemisch in die Reaktionskapillare (4) ein ziehen lässt, und
d) nach Durchtritt des Gemisches durch die Reaktionskapillare (4) das Vorhanden sein oder Fehlen des gesuchten Stoffes durch visuelle Beobachtung des Vorhandenseins bzw. Fehlen von Produkten von Agglutinationsreaktionen im Sammelbereich des Testelementes festgestellt wird.

10. Verwendung nach Anspruch 9 , dadurch gekennzeichnet, dass das Vorhandenseins oder Fehlen eines Kokainmetaboliten bestimmt wird.

## Claims

1. A test element for agglutination tests on a sample-reagent mixture, said test element comprising at least one pair of plate-like members (2, 3) between which a reaction capillary (4) is formed,
the cross-section of the reaction capillary (4) having a size which allows flow of the sample-reagent mixture through the reaction capillary,
the reaction capillary (4) comprising an upstream region (9) and a downstream region (10), an inlet at its upstream end (5) and an outlet at its downstream end (6), and having a substantially rectangular cross-sectional configuration in a direction perpendicular to the longitudinal axis of the reaction capillary, and
the inlet of the reaction capillary (4) being in fluid communication with a region (7) for receiving and mixing the sample with a reagent,
characterized in that
in the upstream region (9) and respectively, in the downstream region (10) the dimension of the reaction capillary (4) in the direction of the thickness increases from the upstream end to the downstream end of each of these regions (9, 10), the increase in this dimension being continuous and linear over the entire length of each of these regions (9, 10).

2. A test element according to claim 1, characterized in that the dimension of the reaction capillary (4) in the direction of the thickness is between 1 and 100 µm at its upstream end (5) and between 5 and 500 µm at its downstream end (6) and in that the smallest dimension of the reaction capillary (4) in the thickness direction in the downstream region (10) is at least equal to the maximum dimension of the reaction capillary (4) in the direction of the thickness in the upstream region (9).

3. A test element according to claim 2, characterized in that the dimension of the reaction capillary (4) in the direction of the thickness is between 50 and 100 µm at its upstream end (5) and between 200 and 300 µm at its downstream end (6).

4. A test element according to claim 1, characterized in that the reaction capillary (4) comprises an intermediate region (11) between the upstream region (9) and the downstream region (10), said intermediate region (11) being in fluid communication with the upstream region and with the downstream region, the dimension in the direction of the thickness in the intermediate region (11) increasing continuously and linearly over the entire length of the intermediate region (11) from its upstream end to its downstream end, and the dimension in the direction of the thickness at the upstream end of the intermediate region (11) being at least equal to the dimension in the direction of the thickness at the downstream end of the upstream region (9), and the dimension in the direction of the thickness at the downstream end of the intermediate region (11) being at maximum equal to the dimension in the direction of the thickness at the upstream end of the downstream region (10).

5. A test element according to claim 1, characterized in that each of the plate-like members (2, 3) has a specific length and the reaction capillary (4) has a length which is greater than the length of one of the plate-like members.

6. A test element according to claim 1, characterized in that the reaction capillary (4) is substantially of S-shaped configuration.

7. A test element according to any one of the preceding claims, characterized in that it also contains a collecting region (8) in fluid communication with the outlet (6) of the reaction capillary (4).

8. A test element according to any one of the preceding claims, characterized in that it is assembled from injection-moulded parts made from a transparent plastic material.

9. The use of a test element according to any one of claims 1 to 8 for immunological tests for the detection of substances, such as drugs, habit-forming substances or the like, or a metabolite of the substance under investigation in body fluids, characterized by
a) introducing given quantities of the body fluid, an antibody reagent and a latex reagent successively into the receiving and mixing region (7) of the test element,
b) mixing the body fluid, the antibody reagent and the latex reagent together so as to form a sample-reagent mixture,
c) allowing the sample-reagent mixture to enter the reaction capillary (4) and
d) after the mixture has travelled through the reaction capillary (4), detecting the presence or absence of the substance under investigation by visual observation of the presence or absence of agglutination-reaction products in the collecting region of the test element.

10. The use according to claim 9, characterized in that the presence or absence of a cocaine metabolite is detected.

## Revendications

1. Elément d'essai pour études d'agglutination d'un mélange échantillon-réactif, lequel élément d'essai contient au moins une paire de corps en forme de plaque (2, 3), entre lesquels est formé un capillaire de réaction (4),
la section transversale du capillaire de réaction (4) ayant une taille permettant l'écoulement de mélange échantillon-réactif dans le capillaire de réaction,
le capillaire de réaction (4) comportant une zone du côté d'arrivée (9) de courant et une zone du côté de sortie (10) de courant, comportant une entrée à son extrémité d'arrivée (5) du courant et une sortie à son extrémité de sortie (6) du courant et ayant une configuration de section transversale pratiquement rectangulaire dans une direction perpendiculaire à l'axe longitudinal du capillaire de réaction, et
l'entrée du capillaire de réaction (4) étant en connexion hydraulique avec une zone (7) pour l'absorption et le mélange de l'échantillon avec un réactif,
caractérisé en ce que
dans la zone du côté d'arrivée (9) du courant ou dans la zone du côté de sortie (10) du courant, la dimension du capillaire de réaction (4) augmente dans le sens de l'épaisseur, de l'extrémité d'arrivée du courant à l'extrémité de sortie du courant de chacune de ces zones (9, 10), l'augmentation de cette dimension étant constante et linéaire sur toute la longueur de chacune de ces zones (9, 10).

2. Elément d'essai selon la revendication 1, caractérisé en ce que, dans le sens de l'épaisseur, la dimension du capillaire de réaction (4) est comprise entre 1 et 100 µm à son extrémité d'arrivée (5) du courant et entre 5 et 500 µm à son extrémité de sortie (6) du courant, et en ce que, dans le sens de l'épaisseur, la plus petite dimension du capillaire de réaction (4) dans la zone du côté de sortie (10) du courant est au moins aussi grande que la plus grande dimension, dans le sens de l'épaisseur, du capillaire de réaction (4) dans la zone du côté d'arrivée (9) du courant.

3. Elément d'essai selon la revendication 2, caractérisé en ce que, dans le sens de l'épaisseur, la dimension du capillaire de réaction (4) est comprise entre 50 et 100 µm à son extrémité d'arrivée (5) du courant et entre 200 et 300 µm à son extrémité de sortie (6) du courant.

4. Elément d'essai selon la revendication 1, caractérisé en ce que, entre la zone du côté d'arrivée (9) du courant et la zone du côté de sortie (10) du courant, le capillaire de réaction (4)) comporte une zone intermédiaire (11) qui est en connexion hydraulique avec la zone du côté d'arrivée du courant et la zone de sortie du courant, dans la zone intermédiaire (11) la dimension dans le sens de l'épaisseur augmentant de façon constante et linéaire sur toute la longueur de la zone intermédiaire (11), de son extrémité d'arrivée du courant à son extrémité de sortie du courant, et, à l'extrémité d'arrivée du courant de la zone intermédiaire (11), la dimension dans le sens de l'épaisseur étant au moins aussi grande que la dimension dans le sens de l'épaisseur à l'extrémité de sortie du courant de la zone du côté d'arrivée (9) du courant, et, à l'extrémité de sortie du courant de la zone intermédiaire (11), la dimension dans le sens de l'épaisseur étant au plus aussi grande que la dimension dans le sens de l'épaisseur à l'extrémité d'arrivée du courant de la zone du côté de sortie (10) du courant.

5. Elément d'essai selon la revendication 1, caractérisé en ce que chacun des corps en forme de plaque (2, 3) a une longueur déterminée et le capillaire de réaction (4) a une longueur qui est supérieure à la longueur d'un des corps en forme de plaque.

6. Elément d'essai selon la revendication 1, caractérisé en ce que le capillaire de réaction (4) a essentiellement un parcours en forme de S.

7. Elément d'essai selon l'une des revendications précédentes, caractérisé en ce qu'il contient en outre une zone collectrice (8) qui est en connexion hydraulique avec la sortie (6) du capillaire de réaction (4).

8. Elément d'essai selon l'une des revendications précédentes, caractérisé en ce qu'il est composé de parties moulées par injection qui sont constituées d'une matière plastique transparente.

9. Utilisation d'un élément d'essai selon l'une des revendications 1 à 8 pour études immunologiques pour la détection de substances, telles que médicaments, drogues ou similaires, ou d'un métabolite de la substance recherchée, dans des liquides corporels, caractérisée en ce que
a) on applique successivement sur la zone d'absorption et de mélange (7) de l'élément d'essai des quantités déterminées du liquide corporel, d'un réactif de type anticorps et d'un réactif de type latex,
b) on mélange entre eux le liquide corporel, le réactif de type anticorps et le réactif de type latex, de manière que ceux-ci forment un mélange échantillon-réactif,
c) on fait passer le mélange échantillon-réactif dans le capillaire de réaction (4), et
d) après passage du mélange dans le capillaire de réaction (4), on détermine la présence ou l'absence de la substance recherchée par examen visuel de la présence ou de l'absence de produits de réactions d'agglutination dans la zone collectrice de l'élément d'essai.

10. Utilisation selon la revendication 9, caractérisée en ce que l'on détermine la présence ou l'absence d'un métabolite de la cocaïne.
